# EUROPEAN PATENT SPECIFICATION

(11) **EP 0 571 671 B1**
(45) Date of publication and mention of the grant of the patent: **17.09.1997**
(21) Application number: 92304712.0
(22) Date of filing: 26.05.1992
(51) Int. Cl.: A61K 9/00

(54) **A Pharmaceutical composition in powder form**
Pulverförmige pharmazeutische Zusammensetzung
Composition pharmaceutique sous forme de poudre

(43) Date of publication of application: 01.12.1993
(73) Proprietor: THE PROCTER & GAMBLE COMPANY, Cincinnati, Ohio 45202 (US)
(72) Inventor: Koochaki, Patricia Elaine, Cincinnati, Ohio 45242 (US)
(74) Representative: Brooks, Maxim Courtney

(56) References cited:
- EP-A- 0 023 359
- DATABASE WPIL Derwent Publications Ltd., London, GB; AN 87061172

## Description

This invention relates to a pharmaceutical composition in powder form for application to the nasal mucosa. In particular, it relates to a sustained-release pharmaceutical composition in powder form comprising a drug, a nonionic cellulose ether derivative and a chitin-derived polymer which provides a high availability of the active ingredient, improved adhesion to the nasal mucosa, solves the common problem of "roll-back" commonly associated with spray and drop formulations such as decongestants and prevents the nasty aftertaste of the formulation active ingredient.

Various pharmaceutical preparations for application to the nasal cavity such as nasal ointments, jellies, nose drops and sprays are known in the art. Nasal ointments and jellies are unsatisfactory, however, because it is difficult to apply them to deep parts of the nasal cavity, such as the concha nasalis superior. Nose drops and sprays have the disadvantage, moreover, that it is difficult to retain the active drugs contained therein in the nasal cavity for an extended period of time. In addition, they are not efficient sustained-release formulations.

In the prior art, attempts have been made to prepare sustained-release formulations for application to the nasal mucosa. US-A-4,226,848 discloses a sustained-release pharmaceutical composition for application to the nasal mucosa containing a drug and a carrier. Sustained-release is achieved by a mechanism in which the pharmaceutical preparation adheres to the nasal mucosa, absorbs the mucus and gradually swells while adhering to the mucosa, and gradually the drug is released from the swollen portion. The composition comprises a mucosa-adhesive polymeric matrix comprising from 50 % to 95 % by weight of a cellulose ether and 50 % to 95 % by weight of a polymer of acrylic acid.

EP-A-0,023,359 discloses a sustained-release, powdery pharmaceutical composition for application to the mucosa of the nasal cavity, comprising a drug and a carrier in which at least 90 % of the composition consists of particles having an effective particle diameter of 20 to 250 µm. The composition comprises a lower alkyl ether of cellulose having a viscosity of 5 to 5000 mPa.s and a drug.

Database WPIL, Derwent Publn. Ltd., AN87061172 discloses a sustained release drug composition in the form of a powder for nasal administration comprising a drug, 75 pts.wt. of carboxymethyl chitin and and 12 pts.wt. of hydroxypropyl cellulose.

Chitin is widely distributed in nature. It is found in tissue support of crustaceans and insects and chitosan is the deacetylation product thereof. Chitin and chitosan have previously been used in pharmaceutical sustained-release preparations. EP-A-187,703 and US-A-4,814,176 disclose a sustained-release preparation in which a combination of chitin and/or chitosan and an anionic polymer is utilised as a sustained-releasing agent.

It is an object of the present invention to provide a sustained-release powdery pharmaceutical composition with improved adhesion characteristics which provides a high availability of the active ingredient and which solves the problem of "roll-back" associated with nasal sprays.

### Summary of the Invention

According to one aspect of the present invention, there is provided a pharmaceutical composition in the form of a powder for application to the mucosa of the nasal cavity and which comprises a drug and a pharmaceutically-acceptable carrier, the carrier comprising
a) from 90 % to 10 % by weight thereof of hydroxypropylmethyl cellulose and
b) from 10 % to 90 % by weight thereof of a chitin-derived polymer selected from chitosan and salts and mixtures thereof;
and wherein the total amount of hydroxypropylmethyl cellulose and chitin-derived polymer comprises at least 50 % by weight of the composition.

### Detailed Description of the Invention

In accordance with the present invention a powdery pharmaceutical composition is formed comprising a drug and a carrier. The carrier comprises a non-ionic cellulose ether derivative and a chitin-derived polymer.

The cellulose ether derivative is present in an amount of 90 % to 10 %, preferably from 85 % to 45 %, by weight of the carrier. The cellulose ether derivative used herein is hydroxypropylmethyl cellulose (HPMC). HPMC is well known in the pharmaceutical and food industries as a thickener and suspending agent. It has substantially no odour or irritation associated with it and is therefore preferred for application to the nasal mucosa which is particularly sensitive to odour and irritation. Furthermore, HPMC easily absorbs mucous from the nasal mucosa, thereby giving the pharmaceutical composition effective adhesiveness and flowability on the nasal mucosa. Whilst a variety of grades of the cellulose ether derivative powders may be used it is preferable that a grade of powder should be used which has a viscosity of from 1 to 50,000 mPa.s, preferably from 5 to 5000 mPa.s (measured at 37°C ± 0.2°C for a 2 % aqueous solution of the cellulose ether derivative), in order that the pharmaceutical composition of the present invention forms a viscous, flowable liquid on application to the nasal mucosa.

The chitin-derived polymer in the carrier is present in an amount from 10 % to 90 %, preferably from 15 % to 55 %, by weight of the carrier. The chitin-derived polymer used herein is selected from chitosan and salts, and mixtures thereof. Chitin is derived from naturally occuring substances such as in tissues of crustaceans and insects and chitosan is the deacetylation product thereof. The formula of chitin is (1->4)-2- acetamide-2-deoxy-β-O-glucan having the following structure:

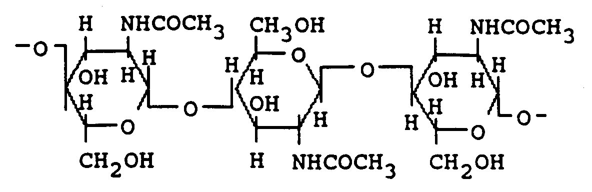

Chitosan has the formula (1->4)-2- amino-2-deoxy-β-D-glucan, and has the following structure:

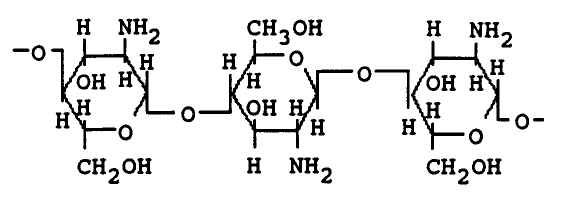

According to the present invention, the preferred chitin-derived polymer is chitosan hydrochloride salt.

Drugs suitable for use in the compositions herein can be selected appropriately according to the disease to which the composition is to be applied. In the present invention the drug is preferably an agent for treating or preventing a nasal disease, preferably a vasoconstrictor or other nasal decongestant. Furthermore the drug should not react with either the cellulose ether derivative or the chitin-derived polymer. Suitable drugs may be in solid or liquid form, preferably solid form.

Examples of such drugs include antipyretic and analgesic agents, antiphlogistics, antiarrhythmics, hypotensors, vasodilators, anticholinergics, antiarteriosclerotics, agents for circulatory systems, antitussives, expectorants, ulcer preventives, enzyme preparations, anti-malignants, chemotherapeutic agents, antihistamine agents, enzyme preparations, local anaesthetic agents, and mouth disinfection agents, steroidal anti-inflammatory agents, non-steroidal anti-inflammatory agent, anti-allergic agents, vasoconstrictors, and mixtures thereof.

In compositions to be used for treating or preventing nasal diseases, drugs effective for treatment or prevention of nasal diseases, such as anti-inflammatory agents, antihistamine agents, anticholinergics, anti-allergic agents or vasoconstrictors, are preferred. A highly preferred drug for inclusion in the composition of the present invention is a vasoconstrictor.

Suitable vasoconstrictors for use herein include phenylephrine hydrochloride, ephedrine hydrochloride, tetrahydrozoline hydrochloride, naphthazoline nitrate, oxymetazoline hydrochloride, xylometazoline hydrochloride and tramazoline hydrochloride, preferably oxymetazoline hydrochloride.

In the pharmaceutical composition of the present invention, it is preferred that at least 85 % by weight of the entire particles should have particle size in the range from 5 to 250 µm, preferably from 10 to 120 µm. This particular range is chosen such that a higher proportion of powder composition adheres to the nasal mucosa when applied to the nasal cavity.

The desired particulate form and particle size can be achieved by spray drying a solution of the pharmaceutical composition ingredients. Of all the industrial dryer types available, spray drying is unique in being able to produce powders of specific particle size and moisture content irrespective of dryer capacity and product heat sensitivity. Spray drying also ensures uniform distribution of the active agent and the carrier. Spray drying consists of four process stages: 1) atomization of feed into a spray, 2) spray-air contact, 3) drying of spray and 4) separation of dry product from the air. Each stage is carried out according to dryer design and operation, and together with the physical and chemical properties of the feed determines the characteristics of the dried product. Spray drying techniques are well known in the art and can be applied in preparing the composition of the invention in known manner.

The present invention is illustrated by the following examples.

### Example 1

A powdery pharmaceutical composition of the invention is prepared as follows:
A stoichiometric quantity of chitosan is mixed with purified water and the pH is adjusted to about 4.5 with 37 % hydrochloric acid. The mixture is stirred for about 2 hours until dissolved. The viscosity of the mixture is then adjusted to 550 cps by the addition of water. Stoichiometric quantities of oxymetazoline hydrochloride and hydroxypropylmethyl cellulose are added with stirring until dissolution is complete. The final solution is filtered by passing it through a centre sieve with a 100 µm filter and the resulting mixture is spray dried at a temperature of 105°C and a minimum pump pressure of 200 bar. In order to achieve the required particle size the powder is passed through a 70 mesh sieve. The final composition of the dry, powdery pharmaceutical composition is as follows:
11 % water
43.5 % chitosan hydrochloride salt
43.5 % hydroxypropylmethyl cellulose
2.4% oxymetazoline hydrochloride.

### Example 2

A second pharmaceutical composition is prepared using the same method as described in Example 1 having the final dry composition:
11 % water
17.5 % chitosan hydrochloride salt
69.5 % hydroxypropylmethyl cellulose
2.4 % oxymetazoline hydrochloride.

The powdery pharmaceutical compositions of the above examples demonstrate improved adhesion characteristics and provide a high availability of active ingredient.

## Claims

1. A pharmaceutical composition in the form of a powder for application to the mucosa of the nasal cavity and which comprises a drug and a pharmaceutically-acceptable carrier, the carrier comprising
a) from 90 % to 10 % by weight thereof of hydroxypropylmethyl cellulose and
b) from 10 % to 90 % by weight thereof of a chitin-derived polymer selected from chitosan, and salts and mixtures thereof;
and wherein the total amount of hydroxypropylmethyl cellulose and chitin-derived polymer comprises at least 50 % by weight of the composition.

2. A pharmaceutical composition according to claim 1 having a particle size distribution such that at least 85 % by weight thereof has a particle size in the range from 5 to 250 µm, preferably from 10 to 120 µm.

3. A pharmaceutical composition according to claim 1 or 2, wherein the drug is at least one member selected from antipyretic and analgesic agents, antiphlogistics, antiarrhythmics, hypotensors, vasodilators, anticholinergics, antiarteriosclerotics, agents for circulatory systems, antitussives, expectorants, ulcer preventives, enzyme preparations, anti-malignants, chemotherapeutic agents, antihistamine agents, local anesthetic agents, and mouth disinfection agents, steroidal anti-inflammatory agents, nonsteroidal anti-inflammatory agent, anti-allergic agents, vasoconstrictors, and mixtures thereof.

4. A pharmaceutical composition according to any of claims 1 to 3 wherein the drug is an agent for treating or preventing a nasal disease.

5. A pharmaceutical composition according to any of claims 1 to 4, wherein said drug is a vasoconstrictor.

6. A pharmaceutical composition according to claim 5 wherein the vasoconstrictor is selected from oxymetazoline hydrochloride, xylometazoline hydrochloride, phenylephrine hydrochloride, ephedrine hydrochloride, tetrahydrozoline hydrochloride, naphthazoline nitrate, tramazoline hydrochloride.

7. A pharmaceutical composition according to claim 6 wherein the vasoconstrictor is oxymetazoline.

8. A pharmaceutical composition according to any of claims 1 to 7 wherein the carrier comprises
a) from 85 % to 45 % by weight thereof of hydroxypropylmethyl cellulose, and
b) from 15 % to 55 % by weight thereof of the chitin-derived polymer.

## Patentansprüche

1. Pulverförmige pharmazeutische Zusammensetzung zur Aufbringung auf die Schleimhaut in der Nasenhöhle, mit einem Gehalt an einem Arzneimittel und einem pharmazeutisch annehmbaren Träger, wobei der Träger
a) von 90 % bis 10 Gew.-% an Hydroxypropylmethylzellulose und
b) von 10 % bis 90 Gew.-% an einem von Chitin abgeleiteten Polymer, ausgewählt unter Chitosan und Salzen und Gemischen hievon, umfaßt;
und worin die Gesamtmenge an Hydroxypropylmethylzellulose und von Chitin abgeleitetem Polymer wenigstens 50 Gew.-% der Zusammensetzung ausmacht.

2. Pharmazeutische Zusammensetzung nach Anspruch 1, mit einer solchen Teilchengrößenverteilung, daß wenigstens 85 Gew.-% hievon eine Teilchengröße im Bereich von 5 bis 250 µm, vorzugsweise 10 bis 120 µm aufweisen.

3. Pharmazeutische Zusammensetzung nach Anspruch 1 oder 2, worin das Arzneimittel wenigstens ein unter antipyretischen und analgetischen Mitteln, Antiphlogistica, Antiarrhythmica, Hypotensoren, Vasodilatoren, Anticholinergica, Antiarteriosclerotica, Kreislaufmitteln, Antitussiva, Expektorantien, Geschwürvorbeugemitteln, Enzympräparaten, Antimalignantien, chemotherapeutischen Mitteln, Antihistaminica, Lokalanästhetica, Munddesinfektionsmitteln, steroiden entzündungshemmenden Mitteln, nichtsteroiden entzündungshemmenden Mitteln, Antiallergica, Vasoconstriktoren und Gemischen hievon ausgewähltes Mittel ist.

4. Pharmazeutische Zusammensetzung nach einem der Ansprüche 1 bis 3, worin das Arzneimittel ein Mittel zur Behandlung oder Verhütung einer Nasenerkrankung ist.

5. Pharmazeutische Zusammensetzung nach einem der Ansprüche 1 bis 4, worin das Arzneimittel ein Vasoconstriktor ist.

6. Pharmazeutische Zusammensetzung nach Anspruch 5, worin der Vasoconstriktor ausgewählt ist unter Oxymetazolinhydrochlorid, Xylometazolinhydrochlorid, Phenylephrinhydrochlorid, Ephedrinhydrochlorid, Tetrahydrozolinhydrochlorid, Naphthazolinnitrat, Tramazolinhydrochlorid.

7. Pharmazeutische Zusammensetzung nach Anspruch 6, worin der Vasoconstriktor Oxymetazolin ist.

8. Pharmazeutische Zusammensetzung nach einem der Ansprüche 1 bis 7, worin der Träger
a) von 85 bis 45 Gew.-% an Hydroxypropylmethylzellulose und
b) von 15 % bis 55 Gew.-% an von Chitin abgeleitetem Polymer umfaßt.

## Revendications

1. Composition pharmaceutique sous forme d'une poudre pour application sur les muqueuses de la cavité nasale, et qui comprend un médicament et un excipient acceptable d'un point de vue pharmaceutique, l'excipient comprenant :
a) de 90 à 10 % en poids, par rapport à l'excipient, d'hydroxypropylméthylcellulose, et
b) de 10 à 90 % en poids, par rapport à l'excipient, d'un polymère dérivé de la chitine, choisi parmi le chitosane et ses sels et mélanges ;
et dans laquelle la quantité totale d'hydroxypropylméthylcellulose et de polymère dérivé de la chitine représente au moins 50 % en poids de la composition.

2. Composition pharmaceutique selon la revendication 1, ayant une distribution granulométrique telle qu'au moins 85 % en poids de cette composition ait une granulométrie comprise entre 5 et 250 µm, de préférence entre 10 et 120 µm.

3. Composition pharmaceutique selon la revendication 1 ou 2, dans laquelle le médicament est au moins un agent choisi parmi les agents antipyrétiques et analgésiques, les antiphlogistiques, les antiarythmiques, les hypotenseurs, les vasodilatateurs, les anticholinergiques, les antiartériosclérotiques, les agents pour les systèmes circulatoires, les antitussifs, les expectorants, les agents de prévention de l'ulcère, les préparations d'enzymes, les antitumoraux, les agents chimiothérapeutiques, les agents antihistaminiques, les agents anesthésiques locaux et les agents de désinfection de la bouche, les agents anti-inflammatoires stéroïdiens, les agents anti-inflammatoires non-stéroïdiens, les agents antiallergiques, les vasoconstricteurs, et leurs mélanges.

4. Composition pharmaceutique selon l'une quelconque des revendications 1 à 3, dans laquelle le médicament est un agent destiné au traitement ou à la prévention d'une maladie nasale.

5. Composition pharmaceutique selon l'une quelconque des revendications 1 à 4, dans laquelle ledit médicament est un vasoconstricteur.

6. Composition pharmaceutique selon la revendication 5, dans laquelle le vasoconstricteur est choisi parmi le chlorhydrate d'oxymétazoline, le chlorhydrate de xylométazoline, le chlorhydrate de phényléphrine, le chlorhydrate d'éphédrine, le chlorhydrate de tétrahydrozoline, le nitrate de naphtazoline, le chlorhydrate de tramazoline.

7. Composition pharmaceutique selon la revendication 6, dans laquelle le vasoconstricteur est l'oxymétazoline.

8. Composition pharmaceutique selon l'une quelconque des revendications 1 à 7, dans laquelle l'excipient comprend :
a) de 85 à 45 % en poids, par rapport à l'excipient, d'hydroxypropylméthylcellulose, et
b) de 15 à 55 % en poids, par rapport à l'excipient, d'un polymère dérivé de la chitine.
